# EUROPÄISCHE PATENTSCHRIFT

(11) **EP 1 417 285 B1**
(45) Veröffentlichungstag und Bekanntmachung des Hinweises auf die Patenterteilung: **04.05.2011**
(21) Anmeldenummer: 02754921.1
(22) Anmeldetag: 22.07.2002
(51) Int. Cl.: C10M 105/38, C07H 13/04, C07H 13/06, C10N 30/02, C10N 40/08

(54) **VERWENDUNG EINER POLYESTER-ZUSAMMENSETZUNG ALS HYDRAULIKFLUID**
USE OF A POLYESTER COMPOSITION AS HYDRAULIC FLUID
UTILISATION D'UNE COMPOSITION DE POLYESTER COMME FLUIDE HYDRAULIQUE

(30) Priorität: 07.08.2001 DE 10138686
(43) Veröffentlichungstag der Anmeldung: 12.05.2004
(73) Patentinhaber: Südzucker Aktiengesellschaft Mannheim/Ochsenfurt, 68165 Mannheim (DE); Fuchs Petrolub AG, 68169 Mannheim (DE)
(72) Erfinder: KUNZ, Markwart, 67550 Worms (DE); KOWALCZYK, Jörg, 67304 Eisenberg/Steinborn (DE); HAJI BEGLI, Alireza, 67305 Ramsen (DE); KOHLSTRUNG, Rainer, 68723 Schwetzingen (DE); HARPERSCHEID, Manfred, 67346 Speyer (DE); KESSELER, Angela, 46047 Oberhausen (DE); LUTHER, Rolf, 67346 Speyer (DE); MANG, Theo, 69469 Weinheim (DE); PUHL, Christian, 67269 Grünstadt (DE); WAGNER, Helena, 50737 Köln (DE)
(74) Vertreter: Schwahn, Hartmut
(86) Internationale Anmeldenummer: PCT/EP2002/008140
(87) Internationale Veröffentlichungsnummer: WO 2003/014269

(56) Entgegenhaltungen:
- EP-A- 0 572 198
- EP-A- 0 879 872
- DE-A- 4 313 948
- GB-A- 793 141
- US-A- 2 700 022
- US-A- 3 468 701
- US-A- 4 144 183
- US-A- 5 102 567

## Beschreibung

Die vorliegende Erfindung betrifft die Verwerdung von Polyestern, die aus einem 1:1 Gemisch von D-Mannit und D-Sorbit, das mit Caprylsäure vollstandig verestert ist, gebildet ist, als Hydraulikflüssigkeit oder Hydraulikfluid.

Hydraulische Antriebe ermöglichen die Übertragung großer Kräfte mit relativ einfachen Elementen und die stufenlose Änderung von Arbeitsgeschwindigkeiten, wobei zwischen dem generatorischen Teil, den Pumpen, und dem motorischen Teil, den Hydromotoren oder Turbinen, über eine Hydraulikflüssigkeit ein hydraulischer Energietransport stattfindet. Entsprechend der Wirkungsweise werden hydrostatische und die weniger häufigen hydrokinetischen Antriebe unterschieden. Der hydrostatische Antrieb arbeitet nach dem Prinzip der Volumenverdrängung in geschlossenen, sich verändernden Räumen. Durch die Pumpe wird pro Hub oder Umdrehung ein bestimmtes Volumen Hydraulikflüssigkeit gegen einen im Strom herrschenden Druck verdrängt (hydromotor). Der hydrokinetische Antrieb wirkt nach dem Trägheitsprinzip durch Umlenken einer in Bewegung befindlichen Hydraulikflüssigkeitsmasse in rotationssymmetrisch angeordneten Schaufelgittern (Turbinen).

Der Hydraulikflüssigkeit kommt bei der Funktionserfüllung hydraulischer Systeme eine wesentliche Bedeutung zu. Grundlegende Aufgaben der Hydraulikflüssigkeit sind die Leistungs- oder Signalübertragung in hydraulischen Systemen. Unter Hydraulikölen oder Hydraulikflüssigkeiten werden daher flüssige Stoffe oder Gemische verstanden, die für die Energieübertragung in hydrostatischen oder hydrokinetischen (hydrodynamischen) Systemen geeignet sind. Neben der Energieübertragung sollen Hydraulikflüssigkeiten für eine ausreichende Schmierung von Tribostellen und für den Schutz der Komponenten des hydraulischen Antriebs vor Korrosion sorgen sowie Wärme aus dem System abführen. Unabhängig von den unterschiedlichen Bedingungen in unterschiedlichen hydraulischen Systemen muss das hydraulische Druckmedium stets eine einwandfreie Funktionsfähigkeit der einzelnen hydraulischen Komponenten garantieren. Neben einem entsprechenden Fließverhalten und guter Kompressibilität, also einer geringen Volumen- und Druckänderung unter Druck, für den störungsfreien Energietransport müssen Hydraulikflüssigkeiten daher möglichst gute Gleiteigenschaften zur Schmierung, hohe spezifische Wärme zur Kühlung, gute Verträglichkeit mit den Anlagen-Werkstoffen sowie korrosionsschützende Eigenschaften aufweisen.

Die verschiedenen hydraulischen Maschinen und Einrichtungen weisen die unterschiedlichsten Betriebsbedingungen auf, beispielsweise extrem hohe oder extrem niedrige Temperaturen. Je nach Anwendung müssen die Hydraulikflüssigkeiten daher nicht nur die vorstehend genannten allgemeinen Funktionseigenschaften besitzen, sondern auch anwendungsspezifische Eigenschaften, die von Fall zu Fall sehr unterschiedlich sein können. Beispielsweise müssen Hydraulikflüssigkeiten für hydraulische Anlagen von Flugzeugen über besonders gute TieftemperaturEigenschaften verfügen. In feuergefährdeten hydraulischen Vorrichtungen, beispielsweise im Steinkohlenbergbau, werden insbesondere schwer entflammbare Hydraulikflüssigkeiten eingesetzt. Die ebenfalls zu den Hydraulikflüssigkeiten gehörenden Bremsflüssigkeiten müssen beispielsweise kälte-, wärme- und alterungs- beziehungsweise oxidationsbeständig, nicht korrodierend und ohne Einfluss auf Gummi sein.

Zu den aus anwendungstechnischer Sicht insbesondere interessierenden spezifischen Kenndaten von Hydraulikflüssigkeiten gehören das Viskositäts-Temperatur-Verhalten, das Viskositäts-Druck-Verhalten und die Bestimmung der Dichte-TemperaturAbhängigkeit. Die Veränderung der Viskosität mit dem Druck beziehungsweise der Temperatur ist für viele herkömmliche hydraulische Flüssigkeiten, insbesondere aus der Gruppe der Mineralöle, gut bekannt. Bei steigender Temperatur weisen beispielsweise als Hydraulikflüssigkeit verwendete Mineralöle deutlich geringere Viskositäten auf als bei niedrigeren Temperaturen. Wird durch zu hohe Temperaturen eine untere Mindestviskosität unterschritten, kommt es in hydraulischen Komponenten zu Misch- beziehungsweise Festkörperreibung, wodurch die Reibung insgesamt erhöht und der Verschleiß verstärkt wird. Zu hohe Viskositäten dagegen sind vor allem aus energetischer Sicht zu vermeiden.

Ebenso wie andere hydraulische Komponenten unterliegt auch das Hydraulikmedium während der Zeit einer Alterung, die sich in einer Änderung der physikalischen und chemischen Kenngrößen äußert. Das Hydraulikmedium verliert beispielsweise seine guten tribologischen Eigenschaften, wobei sich seine ursprüngliche Aufgabe, nämlich der Schutz der Komponenten vor Korrosion, ins Gegenteil umkehren und es zu einem korrosiven Angriff auf die Komponenten des hydraulischen Antriebs kommen kann.

Pro Jahr werden in Deutschland etwa 160.000 Tonnen Hydraulikflüssigkeits-Produkte eingesetzt, wobei etwa 40 % der Hydraulikflüssigkeits-Gesamtmenge auf mobile Anwendungen und 60 % auf stationäre Anwendungen entfallen. Dabei handelt es sich insbesondere um die Hydrauliköle H (alterungsbeständig ohne Wirkzusätze), HL (mit Wirkstoffen zur Erhöhung der Alterungsbeständigkeit und des Korrosionsschutzes), HLP (zusätzlich mit Wirkstoffen zur Verminderung des Verschleißes in Mischreibungsgebiet) sowie HVLP (zusätzlich mit Wirkstoffen zur Verbesserung des Viskositäts-Temperatur-Verhaltens). Zu den eingesetzten Hydraulikflüssigkeits-Produkten gehören auch die schwer entflammbaren Hydraulikflüssigkeiten, die insbesondere in feuergefährdeten hydraulischen Vorrichtungen eingesetzt werden und bei denen die Typen HFA (Öl-in-Wasser-Emulsionen), HFB (Wasser-in-Öl-Emulsionen), HFC (wässrige Polymerlösungen, beispielsweise aus Polyglykolen) sowie HFD (wasserfreie Flüssigkeiten, beispielsweise Phosphorsäureester, Kieselsäureester, Silicone, Halogerkohlenwasserstoffe und andere) unterschieden werden. Ebenfalls dazu gehören Bremsflüssigkeiten, die in hydraulischen Bremssystemen von Fahrzeugen zur Weiterleitung des Bremsdruckes dienen und herkömmlicherweise aus Glykolen, Glykolethern und/oder Polyalkylglykolen bestehen.

Nach einer Erhebung des Umweltbundesamtes (1997) werden im industriellen Bereich 73,2 % der eingesetzten Hydraulikflüssigkeiten nach Verwendung wieder gesammelt und einer Wiederverwertung zugeführt. Das bedeutet, das etwa 30 % aller Hydraulikflüssigkeiten in der Umwelt verbleiben. Dabei gelangt ein großer Teil dieser Produkte vor allem durch Leckagen aus hydraulischen Systemen oder durch Tropfverluste, beispielsweise beim Wechsel von Hydraulikschläuchen bei Baggern, in die Umwelt, in Böden oder Grund- und Oberflächengewässer. Aufgrund ihrer chemischen Zusammensetzung werden viele Hydraulikflüssigkeiten durch die natürlichen Systeme der Umwelt, beispielsweise Mikroorganismen, jedoch kaum oder nur sehr langsam abgebaut. Diese stete Verunreinigung des Erdreiches, der Grund- und Oberflächengewässer mit Hydraulikflüssigkeiten führt daher langfristig zu erheblichen Gefahren für Flora, Fauna und den Menschen.

Vor dem Hintergrund der Gefahren für die Umwelt, die insbesondere von auf Mineralölen basierenden Hydraulikflüssigkeiten ausgehen, werden seit geraumer Zeit Anstrengungen zur Entwicklung von biologisch abbaubaren Hydraulikölen unternommen. Beispielsweise wurden Hydrauliköle entwickelt, die auf pflanzlichen Ölen, wie Rapsöl oder Sonnenblumenöl, und deren Derivaten basieren. Diese auf natürlichen, insbesondere pflanzlichen Ölen basierenden Produkte bieten zwar den Vorteil einer relativ raschen biologischen Abbaubarkeit, besitzen jedoch häufig nicht oder in nur ungenügendem Maße die zur Verwendung als Hydraulikflüssigkeit erforderlichen Eigenschaften, wie Viskositäts-Temperaturverhalten, Langzeit-Kältestabilität, Alterungsstabilität usw.

Weitere nachwachsende Rohstoffe wie Zucker und Stärke blieben bisher für die Anwendung als Hydraulikflüssigkeit ungenutzt und ihr Potential als Polyol-Bestandteil für Synthesester ist nahezu unerforscht. Durch ihre Verfügbarkeit sind solche Rohstoffe jedoch sehr attraktiv, zumal sie durch ihren natürlichen Ursprung Vorteile im Hinblick auf eine schnelle biologische Abbaubarkeit und Umweltverträglichkeit mit sich bringen. Aus dem Stand der Technik sind lediglich einige Anwendungen von Zucker-Verbindungen im Schmiermittel-Bereich, nicht jedoch im Hydraulikflüssigkeits-Bereich bekannt.

Aus der EP 0 879 872 A1 sind biologisch abbaubare, nicht-toxische Schmieröl-Formulierungen bekannt, die aus einem Ester eines Zuckers und einer Fettsäure bestehen. Der Polyol-Bestandteil des Polyesters kann einen Zucker, Zuckeralkohol oder ein Gemisch davon umfassen. Die beschriebene nicht-toxische Schmieröl-Formulierung soll insbesondere Anwendung in Aggregaten finden, die in der Agrar- und Nahrungsmittelindustrie beziehungsweise in der Kosmetik- oder pharmazeutischen Industrie eingesetzt werden.

Die EP 0 572 198 A1 beschreibt SchmierölZusammensetzungen, die ebenfalls für Maschinen zur Herstellung von Nahrungsmitteln eingesetzt werden können. Die Zusammensetzungen umfassen ein Gemisch eines ersten Esters einer mittellangen gesättigten Fettsäure mit Glycerin (Komponente A) und eines zweiten Esters einer Carbonsäure mit Saccharose.

Die DE 42 29 383 C2 beschreibt ein genießbares Schmiermittel mit Zusatz von schmierwirkungsverbessernden Estern aus Fettsäuren und höheren Alkoholen. Die Additive zur Verbesserung der Schmierwirkung bestehen dabei aus mindestens zwei Estern von genießbaren Alkoholen mit mindestens zwei AlkoholGruppen. Als Alkohole können beispielsweise Glycerin, Pentaerythrit, Arabit, Mannit und Sorbit eingesetzt werden.

Die GB 793 141 Bbeschreibt partielle Kohlenhydratcarbonsäureester als Zusatzstoff in Wasser-in-Öl Hydraulikfluiden zur Emulsionstabilisierung.

Das der vorliegenden Erfindung zugrunde liegende technische Problem besteht also darin, vollständig auf der Basis nachwachsender Rohstoffe aufgebaute Syntheseester, die insbesondere unter Verwendung von niedermolekularen Zuckern und aus pflanzlichen Quellen isolierbaren Fettsäuren erhältlich sind, als Basisflüssigkeit für Hydraulikflüssigkeiten bereitzustellen, wobei diese Syntheseester einerseits über die erforderlichen anwendungstechnischen Eigenschaften, wie Oxidations- und Alterungsbeständigkeit, thermische Beständigkeit, geeignetes Viskositäts-Temperatur-Verhalten, geeignetes Viskositäts-Druck-Verhalten etc. verfügen und andererseits aufgrund ihres natürlichen Ursprungs biologisch schnell abbaubar und somit in hohem Maße umweltverträglich sind.

Die Erfindung löst die ihr zugrundeliegende technische Aufgabe durch die Verwendung einer biologisch schnell abbaubaren Zusammensetzung, umfassend ein Polyestergemisch gemäß Anspruch 1 als hydraulisches öl.

Der in der Zusammensetzung enthaltene Polyester oder Zuckerester wird durch die chemische Verknüpfung des Kohlenhydrates und der Carbonsäure beziehungsweise des Carbonsäure-Derivates oder eines Gemisches davon erhalten. Die meisten Zuckerester gehören zur Klasse der nicht-ionischen Tenside. Wegen ihres amphiphilen Charakters, ihrer besonders leichten biologischen Abbaubarkeit und ihrer guten Oberflächeneigenschaften werden diese Zuckerester bisher hauptsächlich in der Lebensmittelindustrie, der Kosmetik und der Pharmazie eingesetzt. Die vorliegende Erfindung stellt daher erstmalig die Verwendung solcher Zuckerester als Basisflüssigkeit von Hydraulikölen bereit. Erfindungsgemäß ist insbesondere vorgesehen, dass der Polyester oder Zuckerester vollständig auf der Basis nachwachsender Rohstoffe, insbesondere heimischer Rohstoffe, beispielsweise Pflanzenöle und Fette, aufgebaut ist. Das heißt, alle Bestandteile, die zur Synthese des erfindungsgemäß als Hydraulikflüssigkeit verwendeten Polyesters eingesetzt werden, also sowohl Kohlenhydrat-Bestandteil als auch Carbonsaure-Bestandteil werden aus nachwachsenden, insbesondere pflanzlichen Rohstoffen, beispielsweise Pflanzenölen und Fetten, gewonnen. Der so aufgebaute Polyester kann dann, wenn er in die Umwelt, beispielsweise in Böden oder Oberflächen- oder Grundwasser gelangt, durch natürliche Systeme wie Mikroorganismen aufgrund seiner natürlichen Bestandteile schnell abgebaut werden.

Untersuchungen der Erfinder der vorliegenden Erfindung haben gezeigt, dass der erfindungsgemäß verwendete Polyester hervorragende Hydraulikflüssigkeits-Eigenschaften, beispielsweise für diesen Anwendungsbereich ausgezeichnet geeignetes Viskositätsverhalten, Lasttragevermögen, Verschleißverhalten, sehr gutes Luftabscheidevermögen und sehr gute oxidative Alterungsbeständigkeit aufweist.

Im Zusammenhang mit der vorliegenden Erfindung bedeutet der Begriff "Verwendung als Hydrauliköl" , "Verwendung als Hydraulikfluid" beziehungsweise "Verwendung als Hydraulikflüssigkeit", dass ein Stoff oder Stoffgemisch, der/das entweder von Natur aus flüssig ist oder der/das nach Lösen in einem flüssigen Medium in flüssiger Form vorliegt, solche Eigenschaften aufweist, die eine Verwendung des Stoffes in hydrostatischen oder hydrokinetischen (hydrodynamischen) Systemen zur Energieübertragung ermöglichen. "Verwendung als Hydraulikflüssigkeit" bedeutet erfindungsgemäß insbesondere die Verwendung als Basisflüssigkeit für Hydrauliköl und schließt die Zugabe weiterer herkömmlicherweise verwendeter Additive für Hydrauliköle, wie phenolischer und/oder aminischer Antioxidantien, Phosphor/Schwefel-Extrem pressure/Antiwear-Zusätze, Korrosionsinhibitoren, Schauminhibitoren und anderer leistungssteigernder Additive nicht aus.

Im Zusammenhang mit der vorliegenden Erfindung bedeutet "biologisch schnell abbaubar", dass die erfindungsgemäß verwendete Polyester-Zusammensetzung durch die biologischen Systeme der Umwelt, insbesondere in der Umwelt vorhandene Mikroorganismen wie Bakterien und Pilze, rasch abgebaut wird. Dabei stellen auch die entstehenden niedermolekularen Abbauprodukte keine Umweltbelastung dar, entweder weil es sich bereits um natürlicherweise vorkommende Substanzen handelt, die für Flora und Fauna ungiftig und somit umweltverträglich sind, oder weil die Abbauprodukte durch nachfolgende biologische Systeme, insbesondere weitere Mikroorganismen, zu solchen natürlicherweise vorkommenden, nichtgiftigen Substanzen abgebaut werden können. Erfindungsgemäß ist also vorgesehen, das der natürliche Abbau der erfindungsgemäß als Hydraulikflüssigkeit eingesetzten Zusammensetzung im wesentlichen zu Produkten führt, die für Lebewesen, wie Tier und Mensch unbedenklich sind.

Gemäß der Erfindung ist vorgesehen, dass alle freien Hydroxyl-Gruppen des Kohlenhydrates mit mindestens einer Carbonsäure, mindestens einem Carbonsäure-Derivat oder einem Gemisch davon verestert sind.

Die Erfindung betrifft die Verwendung eines Kohlenhydratesters als Hydrauliköl, wobei der Polyester herstellbar ist durch Veresterung oder Umesterung des Kohlenhydrates oder eines mehrere verschiedene Kohlenhydrate enthaltenden Gemisches in Lösungsmitteln oder ohne Lösungsmittel in Gegenwart eines Katalysators. Die Produkte, die zur Verwendung als Hydrauliköl oder Hydraulikflüssigkeit vorgesehen sind, können also in bekannten organischen Lösungsmitteln wie Toluol, DMSO, Pyridin, DMF und ähnlichen, aber auch lösungsmittelfrei, durch Veresterung beziehungsweise Umesterung des Kohlenhydrat-(Polyol-)Gemisches mit den entsprechenden Reagenzien unter Zusatz geeigneter Katalysatoren hergestellt werden.

Es ist insbesondere vorgesehen, dass die erfindungsgemäß als Hydraulikflüssigkeit eingesetzten Produkte unter Verwendung von Übergangsmetall-Verbindungen von insbesondere Sn, Ti oder Zn/Cu, beispielsweise Salze, Oxide, Alkyle usw., Mineralsäuren wie HCl, H₂SO₄ oder H₃PO₄, organischen Säuren wie p-Toluolsulfonsäure, Methansulfonsäure oder Sulfobernsteinsäure, sauren Ionenaustauschern, Alkalisalzen wie Hydroxide, Carbonate, Methanolate, Ethanolate von beispielsweise Natrium oder Kalium, Zeolithen oder Gemischen davon hergestellt werden. Erfindungsgemäß besonders bevorzugt ist die Verwendung von p-Toluolsulfonsäure oder eines Zinn-Oxalatkatalysators als Katalysator.

Es ist vorgesehen, dass die zur Verwendung als Hydraulikflüssigkeit vorgesehenen Kohlenhydratester durch eine Umesterung oder Veresterung in einem oder mehreren Lösungsmitten oder ohne Lösungsmittel hergestellt werden. Erfindungsgemäß besonders bevorzugt sind organische Lösungsmittel wie Toluol, DMSO, Pyridin oder DMF.

Es ist vorgesehen, dass die zur Verwendung als Hydraulikflüssigkeit vorgesehenen Kohlenhydratester durch eine Umesterung oder Veresterung bei einer Temperatur von 120°C bis 280°C hergestellt werden. Besonders bevorzugt ist die Verwendung von Kohlenhydratestern, die bei einer Veresterungs- beziehungsweise Umesterungstemperatur von 160°C bis 250°C hergestellt werden.

Das Verhältnis der Kohlenhydrat- und Säure-Ausgangsbestandteile, insbesondere das Verhältnis von Hydroxylgruppen zu Carbonsäuregruppen, bei der Veresterung beziehungsweise Umesterung hat entscheidenden Einfluss auf den Veresterungsgrad der erhaltenen Kohlenhydratester. Die Menge des bei der Es Umsetzung einzusetzenden Säure-Bestandteils, bezogen auf die Menge des eingesetzten KohlenhydratBestandteils, hängt davon ab, wie viele freie Hydroxyl-Gruppen das als Ausgangsmaterial eingesetzte Kohlenhydrat aufweist. Es ist insbesondere vorgesehen, dass das Verhältnis von Hydroxylgruppen zu Carbonsäuregruppen 1:1 bis 1:10 beträgt. Eine bevorzugte Ausgestaltung betrifft daher die Verwendung von Kohlenhydratestern, bei deren Herstellung das Verhältnis von Hydroxylgruppen zu Carbonsäuregruppen der Ausgangsbestandteile bei 1:1 bis 1:10 liegt. Besonders bevorzugt ist die Verwendung von Kohlenhydratestern, bei deren Herstellung das Ausgangs-Verhältnis von Hydroxylgruppen zu Carbonsäuregruppen der Kohlenhydrat- und Säure-Ausgangsbestandteile 1:1,5 bis 1:7 beträgt.

Auch die zur Veresterung oder Umesterung verwendete Reaktionszeit hat entscheidenden Einfluss auf den Veresterungsgrad der erhaltenen Produkte. Es ist vorgesehen, dass die Dauer der Veresterung beziehungsweise Umesterung der Kohlenhydrate mit Carbonsäuren 2 bis 36 Stunden, besonders bevorzugt 4 bis 26 Stunden, am stärksten bevorzugt 8 bis 10 Stunden betragt. Eine bevorzugte Ausgestaltung betrifft daher die Verwendung von Kohlenhydratestern, deren Herstellung durch Veresterung oder Umesterung eines Kohlenhydrates mit einer Carbonsäure oder einem Carbonsäure-Derivat 2 bis 36 Stunden, besonders bevorzugt 4 bis 26 Stunden, am stärksten bevorzugt 8 bis 10 Stunden dauert.

Die bevorzugten Reaktionsbedingungen zur Veresterung oder Umesterung von Kohlenhydraten zur Herstellung der erfindungsgemäß verwendeten Polyester umfassen vorzugsweise die folgenden Parameter: Verwendung eines Rührreaktors, wobei die Veresterung auch zwei- bis fünfstufig in einer Rührkesselkaskade durchgeführt werden kann, Entfernung von Wasser während der Reaktion durch Rektifikation oder Azeotroprektifikation, Durchführung der Reaktion in einem organischen Lösungsmittel, beispielsweise Toluol, DMF oder Ether, oder ohne Lösungsmittel, eine Reaktionsdauer von 2 bis 36 Stunden, vorzugsweise 8 bis 26 Stunden, und Durchführung der Reaktion in Gegenwart eines Katalysators, wobei die Katalysatormenge, bezogen auf die Gesamtmenge, bei 0,05 - 10 Gew.-%, vorzugsweise bei 0,1 - 5 Gew.-% Liegt. Die Kohlenhydrat- und Säure-Ausgangssubstanzen liegen, bezogen auf die Monomereinheiten, vorzugsweise in einem Verhältnis von 1:1 bis 1:10 vor, besonders bevorzugt in einem Verhältnis von 1:1,5 bis 1:7. Die Umsetzung erfolgt vorzugsweise im Vakuum bei 300 bis 10 mbar.

Die erfindungsgemäß zur Verwendung als Hydraulikflüssigkeiten beziehungsweise Hydrauliköle vorgesehenen Kohlenhydratester weisen hervorragende physikalisch-chemische Eigenschaften auf, die sie insbesondere für diesen Anwendungsbereich prädestinieren. Die erfindungsgemäß verwendeten Kohlenhydratester besitzen beispielsweise bei 40°C eine kinematische Viskosität von etwa 20 bis 120 mm²/s. Untersuchungen haben auch ergeben, dass sie eine ausgezeichnete Langzeit-Kältestabilität aufweisen, da sie nach mehreren Tagen bei -25°C noch fließfähig sind. Ihre hervorragende oxidative Alterungsbeständigkeit lässt sich im Turbine Oil Stability-Test nachweisen.

Eine bevorzugte Ausführungsform betrifft daher die Verwendung von Kohlenhydratester-Zusammensetzungen, die eine kinematische Viskosität von 20 bis 120 mm²/s aufweisen, als Hydraulikflüssigkeit. Eine weitere Ausführungsform betrifft die Verwendung von Kohlenhydratestern als Hydraulikflüssigkeit, die eine solche Langzeit-Kältestabilität aufweisen, dass sie noch nach drei Tagen bei einer Temperatur von -25°C fließfähig sind. Noch eine weitere bevorzugte Ausfühhrungsform der Erfindung betrifft die Verwendung einer Kohlenhydratester-Zusammensetzung als Basisflüssigkeit für hydraulische Öle, die dadurch charakterisiert ist, dass sie einen Pourpoint aufweist, der kleiner als -25°C ist. Es ist auch vorgesehen, dass Kohlenhydratester-Zusammensetzungen als Hydraulikflüssigkeit verwendeten werden, die im Testverfahren FZG A/8,3/90 ein Lasttragevermögen von mindestens Laststufe 10 aufweisen. Erfindungsgemäß ist ferner vorgesehen, dass Kohlenhydratester-Zusammensetzungen als Hydraulikflüssigkeit verwendeten werden, die im Turbine Oil Stability-Test ohne Wasserzusatz mehr als 1800 h benötigen, bis eine Säurezahl von 2 mg KOH/g erreicht ist.

Die vorliegende Erfindung wird durch die folgenden Beispiele näher erläutert.

### Beispiel 1

### Herstellung eines Zuckeresters durch Veresterung von D-Sorbit und D-Mannit mit Caprylsäureanhydrid (diskontinuierliche Variante)

In einem Rührreaktor wurden 250 g eines 1:1-Gemisches von D-Sorbit und D-Mannit in Gegenwart von 0,8 g p-Toluolsulfonsäure 1,25 h bei 155°C dehydratisiert. Nach Zugabe von 1,86 kg Caprylsäureanhydrid und 6 g Zinnoxalat wurde das Gemisch 10 h bei 195°C gerührt, wobei Wasser destillativ entfernt wurde. Nach beendeter Reaktion und Entfernung des Katalysators wurde überschüssige Säure im Vakuum entfernt. Als Produkt wurde ein klares hellgelbes Öl erhalten.

### Beispiel 2

### Verwendung des mit Caprylsäureanhydrid vollständig veresterten Produktes als Hydraulikflüssigkeit

Das in Beispiel 1 erhaltene Produkt aus der Veresterungsreaktion von D-Sorbit und D-Mannit mit Caprylsäureanhydrid (n-C8) wurde als Basisflüssigkeit für Hydrauliköle getestet. Das in Beispiel 1 erhaltene Produkt wurde dabei mit typischen Additiven für Hydrauliköle, wie phenolischen und aminischen Antioxidantien, Phosphor/Schwefel-Extrempressure/Antiwear-Zusätzen, Korrosionsinhibitoren und einem Schauminhibitor additiviert. Anschließend wurden die Eigenschaften dieses Gemisches im Hinblick auf seine Eignung als Hydraulikflüssigkeit untersucht. Dabei wurden die folgenden Ergebnisse erhalten:
Kinematische Viskosität bei 40°C: 36 mm²/s
Pourpoint: -30°C. Die Messung erfolgte nach DIN ISO 3016. Dieser Wert ist als gut zu beurteilen.
Langzeitkältestabilität: Nach 3 Tagen bei -25°C noch fließfähig. Dieser Wert ist als gut zu beurteilen.
Luftabscheidevermögen bei 50°C: 3 Minuten. Die Messung erfolgte nach DIN 51381. Dieser Wert ist als gut zu beurteilen.
Demulgiervermögen: 25 Minuten bei 50°C. Die Messung erfolgte nach DIN 51599. Dieser Wert ist als gut zu beurteillen.
Lasttragevermögen/Verschleißverhalten: Laststufe 11 noch schadensfrei im Testverfahren FZG A/8,3/90. Der Verschleißkalottendurchmesser betrug 0,31 mm im Vierkugel-Apparat nach DIN 51350. Die Werte sind als sehr gut zu beurteilen.
Alterungsstabilität: 1.900 Stunden im Turbine Oil Stability-Test ohne Wasserzusatz bis eine Säurezahl von 2 mg KOH/g erreicht ist.

### Vergleichsbeispiel 1

### Testung von vollständig verestertem Glycerin auf Eignung als Hydraulikflüssigkeit

Als Basisflüssigkeit wurde Glycerin eingesetzt, das mit einem Gemisch aus Caprylsäure und Caprinsäure vollständig verestert worden war. Das dabei erhaltene Produkt wurde mit phenolischen und aminischen Antioxydantien, Phosphor/Schwefel-Extrem pressure/Antiwear-Zusätzen, Korrosionsinhibitoren und einem Schauminhibitor additiviert, wobei die Zusätze identisch zu den Zusätzen in Beispiel 2 waren. Dabei wurden für diese Basisflüssigkeit die folgenden Eigenschaften festgestellt:
Kinematische Viskosität bei 40°C: 15 mm²/s. Für die meisten Applikationen ist dieser Wert zu niedrig.
Pourpoint: -10°C. Die Messung erfolgt DIN ISS 3016. Diese= Wert ist für die meisten Applikationen, insbesondere in kälteren Klimaten, nicht niedrig genug.
Langzeit-Kältestabilität Nach 3 Tagen bei -25°C nicht mehr fließfähig. Dieser Wert ist für Anwendungen in kalten Klimaten nicht akzeptabel.
Luftabscheidevermögen bei 50°C: 6 Minuten. Die Messung erfolgte nach DIN 51381. Der Wert ist als mäßig gut zu beurteilen.
Demulgiervermögen: 20 Minuten bei 50°C. Die Messung erfolgte nach DIN 51599. Der Wert ist als gut zu beurteilen.
Lasttragevermögen/Verschleißverhalten: Laststufe 10 noch schadensfrei im Testverfahren FZG A/8,3/90. Verschleißkalottendurchmesser 0,35 mm im Vierkugel-Apparat nach DIN 51350. Beide Werte sind als mäßig gut zu beurteilen.
Alterungsstabilität: 1.200 Stunden in Turbine Oil Stability-Test ohne Wasserzusatz, bis eine Säurezahl von 2 mm KOH/g erreicht wurde. Der Wert ist als mäßig gut zu beurteilen.

### Vergleichsbeispiel 2

### Testung von Glycerin, das mit Sonnenblumenöl-Fettsäure verestert wurde, im Hinblick auf die Eignung als Hydraulikflüssigkeit

Als Basisflüssigkeit wurde Glycerin, das mit Sonnenblumenöl-Fettsäure (high oleic-Qualität, Ölsäureanteil 80 %) war, verwendet. Der Glycerinester wurde mit phenolischen und aminischen Antioxidantien, Phosphor/Schwefel-Extrem pressure/Antiwear-Zusätzen, Korrosionsinhibitoren und einem Schauminhibitor additiviert. Die Zusätze waren dabei identisch zu den in Beispiel 2 verwendeten. Für die so erhaltene Basisflüssigkeit wurden die folgenden Eigenschaften festgestellt:
Kinematische Viskosität bei 40°C: 38 mm²/s.
Pourpoint: -10°C. Die Messung erfolgte nach DIN ISO 3016. Der Wert ist für die meisten Applikationen, insbesondere in kälteren Klimaten, nicht niedrig genug.
Langzeit-Kältestabilität: Nach 3 Tagen bei -25°C nicht mehr fließfähig. Dieser Wert ist für Anwendungen in kalten Klimaten nicht akzeptabel.
Luftabscheidevermögen bei 50°C: 4 Minuten. Die Messung erfolgte nach DIN 5381. Der Wert ist als gut zu beurteilen.
Demulgiervermögen: 22 Minuten bei 50°C. Die Messung erfolgte nach DIN 51599. Der Wert ist als gut zu beurteilen.
Lasttragevermögen/Verschleißverhalten: Höchste Laststufe (12) noch schadensfrei im Testverfahren FZG A/8,3/90. Verschleißkalottendurchmesser 0,31 mm im Vierkugel-Apparat nach DIN 51350. Beide Werte sind als sehr gut zu beurteilen.
Alterungsstabilität: 450 Stunden im Turbine Oil Stability-Test ohne Wasserzusatz, bis eine Säurezahl von 2 mm KOH/g erreicht wurde. Dieser Wert ist als schlecht zu beurteilen.

## Patentansprüche

1. Verwendung von Polyestergemisch, das aus einem 1:1 Gemisch von D-Mannit und D-Sorbit, das mit Caprylsäure vollständig verestert ist, gebildet ist, als Basisflüssigkeit für Hydrauliköl.

2. Verwendung nach Anspruch 1, wobei das PolyesterGemisch mit mindestens einem zur Herstellung von Hydraulikölen geeigneten Additiv ,ausgewählt aus der Gruppe bestehend aus phenolischen oder aminischen Additiven, Phosphor/Schwefel-Extreme pressure/Antiwear-Zusätzen, Korrosionsinhibitoren und Schauminhibitoren, versetzt ist.

## Claims

1. Use of a polyester mixture, which consists of a 1:1 mixture of D-mannitol and D-sorbitol fully estered with carprylic acid as a base fluid for hydraulic oil.

2. Use according to claim 1, **characterized in that** the polyester mixture is mixed with at least one additive suitable for manufacturing hydraulic oils, which is selected from the group consisting of phenolic or aminic additives, phosphor/sulfur extreme pressure/antiwear additives, corrosion inhibitors and foam inhibitors.

## Revendications

1. Utilisation en tant que fluide de base pour une huile hydraulique d'un mélange de polyester, composé d'un mélange 1 : 1 de D-mannitol et de D-sorbitol, qui est totalement estérifié à l'aide d'un acide caprylique.

2. Utilisation selon la revendication 1, le mélange de polyester étant pourvu d'au moins un additif approprié pour la fabrication d'huiles hydrauliques, sélectionné parmi le groupe consistant en un additif phénolique ou aminé, des agents complémentaires anti-usure / extrême pression contenant du phosphore / soufre, des inhibiteurs de la corrosion et des inhibiteurs de mousse.
